# EUROPEAN PATENT APPLICATION

(11) **EP 3 489 358 A1**
(43) Date of publication of application: **29.05.2019**
(21) Application number: 17830737.7
(22) Date of filing: 08.06.2017
(51) Int. Cl.: C12N 13/00, A01H 1/00, C12N 15/09

(54) **METHOD FOR INTRODUCING SUBSTANCE INTO PLANT CELL USING PLASMA**

(30) Priority: 19.07.2016 JP 2016141638
(71) Applicant: National Agriculture and Food Research Organization, Tsukuba-shi, Ibaraki 305-8517 (JP); Tokyo Institute of Technology, Tokyo 152-8550 (JP)
(72) Inventor: MITSUHARA, Ichiro, Tsukuba-shi Ibaraki 305-8602 (JP); YANAGAWA, Yuki, Tsukuba-shi Ibaraki 305-8602 (JP); OKINO, Akitoshi, Tokyo 152-8550 (JP); MIYAHARA ,Hidekazu, Tokyo 152-8550 (JP); KAWANO, Hiroaki, Tokyo 152-8550 (JP); KOBAYASHI, Tomohiro, Tokyo 152-8550 (JP); WATANABE, Yosuke, Tokyo 152-8550 (JP)
(74) Representative: J A Kemp
(86) International application number: PCT/JP2017/021361
(87) International publication number: WO 2018/016217

(57) **Abstract**

It was found that bringing a substance into contact with a plant cell after the plant cell is treated with plasma makes it possible to easily and highly efficiently introduce the substance into the cell without causing a damae regardless of the types of the plant and tissue from which the plant cell is derived.

### [Sequence Listing]

SEQUENCE LISTING

<110>  NATIONAL AGRICULTURE AND FOOD RESEARCH ORGANIZATION
       TOKYO INSTITUTE OF TECHNOLOGY

<120>  METHOD FOR INTRODUCING SUBSTANCE INTO PLANT CELL USING PLASMA

<130>  N415358EP

<140>  EP17830737.7
<141>  2017-06-08

<150>  JP2016-141638
<151>  2016-07-19

<160>  10

<170>  PatentIn version 3.5

<210>  1
<211>  720
<212>  DNA
<213>  Artificial Sequence

<220>
<223>  Superfolder GFP gene sequence

<400>  1
atggtgagca agggcgagga gctgttcacc ggggtggtgc ccatcctggt cgagctggac      60

ggcgacgtga acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccttcaccta cggcgtgcag tgcttcagcc gctaccccga ccacatgaag     240

cagcacgact tcttcaagtc cgccatgccc gaaggctacg tccaggagcg caccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaactacaa cagccacaac gtctatatca tggccgacaa gcagaagaac     480

ggcatcaagg tgaacttcaa gatccgccac aacatcgagg acggcagcgt gcagctcgcc     540

gaccactacc agcagaacac ccccatcggc gacggccccg tgctgctgcc cgacaaccac     600

tacctgagca cccagtccgc cctgagcaaa gaccccaacg agaagcgcga tcacatggtc     660

ctgctggagt tcgtgaccgc cgccgggatc actcacggca tggacgagct gtacaagtaa     720


<210>  2
<211>  30
<212>  DNA
<213>  Artificial Sequence

<220>
<223>  Artificially synthesized primer sequence (BamH1-sGFP-F)

<400>  2
taggattcac catggtgagc aagggcgagg                                       30

<210> 3
<211> 30
<212> DNA
<213> Artificial Sequence

<220>
<223> Artificially synthesized primer sequence (EcoR1-sGFP-R)

<400> 3
tagaattcct tgtacagctc gtccatgccg 30


<210> 4
<211> 5121
<212> DNA
<213> Bordetella pertussis

<220>
<223> Adenylate cyclase

<400> 4
   atgcagcaat cgcatcaggc tggttacgca aacgccgccg accgggagtc tggcatcccc 60
   gcagccgtac tcgatggcat caaggccgtg gcgaaggaaa aaaacgccac attgatgttc 120
   cgcctggtca acccccattc caccagcctg attgccgaag gggtggccac caaaggattg 180
   ggcgtgcacg ccaagtcgtc cgattggggg ttgcaggcgg gctacattcc cgtcaacccg 240
   aatctttcca aactgttcgg ccgtgcgccc gaggtgatcg cgcgggccga caacgacgtc 300
   aacagcagcc tggcgcatgg ccataccgcg gtcgacctga cgctgtcgaa agagcggctt 360
   gactatctgc ggcaagcggg cctggtcacc ggcatggccg atggcgtggt cgcgagcaac 420
   cacgcaggct acgagcagtt cgagtttcgc gtgaaggaaa cctcggacgg gcgctatgcc 480
   gtgcagtatc gccgcaaggg cggcgacgat ttcgaggcgg tcaaggtgat cggcaatgcc 540
   gccggtattc cactgacggc ggatatcgac atgttcgcca ttatgccgca tctgtccaac 600
   ttccgcgact cggcgcgcag ttcggtgacc agcggcgatt cggtgaccga ttacctggcg 660
   cgcacgcggc gggccgccag cgaggccacg ggcggcctgg atcgcgaacg catcgacttg 720
   ttgtggaaaa tcgctcgcgc cggcgcccgt tccgcagtgg gcaccgaggc gcgtcgccag 780
   ttccgctacg acggcgacat gaatatcggc gtgatcaccg atttcgagct ggaagtgcgc 840
   aatgcgctga acaggcgggc gcacgccgtc ggcgcgcagg acgtggtcca gcatggcact 900
   gagcagaaca atcctttccc ggaggcagat gagaagattt tcgtcgtatc ggccaccggt 960
   gaaagccaga tgctcacgcg cgggcaactg aaggaataca ttggccagca gcgcggcgag 1020
   ggctatgtct tctacgagaa ccgtgcatac ggcgtggcgg ggaaaagcct gttcgacgat 1080
   gggctgggag ccgcgcccgg cgtgccgagc ggacgttcga agttctcgcc ggatgtactg 1140
   gaaacggtgc cggcgtcacc cggattgcgg cggccgtcgc tgggcgcagt ggaacgccag 1200
   gattccggct atgacagcct tgatggggtg ggatcgcgat cgttctcgtt gggcgaggtg 1260
   tccgacatgg ccgccgtgga agcggcggaa ctggaaatga cccggcaagt cttgcacgcc 1320
   ggggcgcggc aggacgatgc cgagccgggc gtgagcggtg cgtcggcgca ctgggggcag 1380
   cgggcgctgc agggcgccca ggcggtggcg gcggcgcagc ggctggttca tgccattgcc 1440
   ctgatgacgc aattcggccg ggccggttcc accaacacgc cgcaggaagc ggcctcgttg 1500
   tcggcggccg tgttcggctt gggcgaggcc agcagcgccg tggccgaaac cgtgagcggt 1560
   tttttccgcg ggtcttcgcg ctgggccggc ggtttcggcg tggctggcgg cgcgatggcg 1620
   ctgggaggcg gcatcgccgc ggccgttggc gccgggatgt cgttgaccga tgacgcgccg 1680
   gccggacaga aggccgccgc cggcgccgag atcgcgctgc agttgacagg tggaacggtc 1740
   gagctggctt cttccatcgc gttggcgctg gccgcggcgc gcggcgtgac cagcggcttg 1800
   caggtggccg gggcgtcggc cggggcggct gccggcgcat tggccgcggc gctcagtccc 1860
   atggagatct acggcctggt gcagcaatcg cactatgcgg atcagctgga caagctggcg 1920
   caggaatcga gcgcatacgg ttacgagggc gacgccttgc tggcccagct gtatcgcgac 1980
   aagacggccg ccgagggcgc cgtcgccggc gtctccgccg tcctgagcac ggtgggggcg 2040
   gcggtgtcga tcgccgcggc ggccagcgtg gtagggcccc cggtggcggt ggtcacttcc 2100
   ttgctgaccg gggctctcaa cggcatcctg cgcggcgtgc agcagcccat catcgaaaag 2160
   ctggccaacg attacgctcg caagatcgac gagctgggcg ggccgcaagc gtacttcgag 2220
   aaaaacctgc aggcgcgtca cgaacaactg gccaattcgg acggcctacg gaaaatgctg 2280
   gccgacctgc aggccggttg gaacgccagc agcgtgatcg gggtgcagac gacagagatc 2340
   tccaagtcgg cgctcgaact ggccgccatt accggcaacg cggacaacct gaaatccgtc 2400
   gacgtgttcg tggaccgctt cgtccagggc gagcgggtgg ccggccagcc ggtggtcctc 2460
   gacgtcgccg ccggcggcat cgatatcgcc agccgcaagg gcgagcggcc ggcgctgacg 2520
   ttcatcacgc cgctggccgc gccaggagaa gagcagcgcc ggcgcacgaa aacgggcaag 2580
   agcgaattca ccacattcgt cgagatcgtg ggcaagcagg accgctggcg catccgggac 2640
   ggcgcggccg acaccaccat cgatctggcc aaggtggtgt cgcaactggt cgacgccaat 2700
   ggcgtgctca agcacagcat caaactggat gtgatcggcg gagatggcga tgacgtcgtg 2760
   cttgccaatg cttcgcgcat ccattatgac ggcggcgcgg gcaccaacac ggtcagctat 2820
   gccgccctgg gtcgacagga ttccattacc gtgtccgccg acggggaacg tttcaacgtg 2880
   cgcaagcagt tgaacaacgc caacgtgtat cgcgaaggcg tggctaccca gacaaccgcc 2940
   tacggcaagc gcacggagaa tgtccaatac cgccatgtcg agctggcccg tgtcgggcaa 3000
   gtggtggagg tcgacacgct cgagcatgtg cagcacatca tcggcggggc cggcaacgat 3060
   tcgatcaccg gcaatgcgca cgacaacttc ctagccggcg ggtcgggcga cgacaggctg 3120
   gatggcggcg ccggcaacga caccctggtt ggcggcgagg gccaaaacac ggtcatcggc 3180
   ggcgccggcg acgacgtatt cctgcaggac ctgggggtat ggagcaacca gctcgatggc 3240
   ggcgcgggcg tcgataccgt gaagtacaac gtgcaccagc cttccgagga gcgcctcgaa 3300
   cgcatgggcg acacgggcat ccatgccgat cttcaaaagg gcacggtcga gaagtggccg 3360
   gccctgaacc tgttcagcgt cgaccatgtc aagaatatcg agaatctgca cggctcccgc 3420
   ctaaacgacc gcatcgccgg cgacgaccag gacaacgagc tctgggccca cgatggcaac 3480
   gacacgatac gcggccgggg cggcgacgac atcctgcgcg gcggcctggg cctggacacg 3540
   ctgtatggcg aggacggcaa cgacatcttc ctgcaggacg acgagaccgt cagcgatgac 3600
   atcgacggcg gcgcggggct ggacaccgtc gactactccg ccatgatcca tccaggcagg 3660
   atcgttgcgc cgcatgaata cggcttcggg atcgaggcgg acctgtccag ggaatgggtg 3720
   cgcaaggcgt ccgcgctggg cgtggactat tacgataatg tccgcaatgt cgaaaacgtc 3780
   atcggtacga gcatgaagga tgtgctcatc ggcgacgcgc aagccaatac cctgatgggc 3840
   cagggcggcg acgataccgt gcgcggcggc gacggcgatg atctgctgtt cggcggcgac 3900
   ggcaacgaca tgctgtatgg cgacgccggc aacgacaccc tctacggggg gctgggcgac 3960
   gatacccttg aaggcggcgc gggcaacgat tggttcggcc agacgcaggc gcgcgagcat 4020
   gacgtgctgc gcggcggaga tggggtggat accgtcgatt acagccagac cggcgcgcat 4080
   gccggcattg ccgcgggtcg catcgggctg ggcatcctgg ctgacctggg cgccggccgc 4140
   gtcgacaagc tgggcgaggc cggcagcagc gcctacgata cggtttccgg tatcgagaac 4200
   gtggtgggca cggaactggc cgaccgcatc acgggcgatg cgcaggccaa cgtgctgcgc 4260
   ggcgcgggtg gcgccgacgt gcttgcgggc ggcgagggcg acgatgtgct gctgggcggc 4320
   gacggcgacg accagctgtc gggcgacgcc ggacgcgatc gcttgtacgg cgaagccggt 4380
   gacgactggt tcttccagga tgccgccaat gccggcaatc tgctcgacgg cggcgacggc 4440
   cgcgataccg tggatttcag cggcccgggc cggggcctcg acgccggcgc aaagggcgta 4500
   ttcctgagct tgggcaaggg gttcgccagc ctgatggacg aacccgaaac cagcaacgtg 4560
   ttgcgcaata tcgagaacgc cgtgggcagc gcgcgtgatg acgtgctgat cggcgacgca 4620
   ggcgccaacg tcctcaatgg cctggcgggc aacgacgtgc tgtccggcgg cgctggcgac 4680
   gatgtgctgc tgggcgacga gggctcggac ctgctcagcg gcgatgcggg caacgacgat 4740
   ctgttcggcg ggcagggcga tgatacttat ctgttcgggg tcgggtacgg gcacgacacg 4800
   atctacgaat cgggcggcgg ccatgacacc atccgcatca acgcgggggc ggaccagctg 4860
   tggttcgcgc gccagggcaa cgacctggag atccgcattc tcggcaccga cgatgcactt 4920
   accgtgcacg actggtatcg cgacgccgat caccgggtgg aaatcatcca tgccgccaac 4980
   caggcggtag accaggcagg catcgaaaag ctggtcgagg caatggcgca gtatccggac 5040
   cccggcgcgg cggcggctgc cccgccggcg gcgcgcgtgc cggacacgct gatgcagtcc 5100
   ctggctgtca actggcgctg a 5121

<210> 5
<211> 28
<212> DNA
<213> Artificial Sequence

<220>
<223> Artificially synthesized primer sequence (EcoR1-Cya-F)

<400> 5
tagaattcat gcagcaatcg catcaggc 28

<210> 6
<211> 31
<212> DNA
<213> Artificial Sequence

<220>
<223> Artificially synthesized primer sequence (Xho1-Stop-Cya1200R)

<400> 6
tcactcgagc tactggcgtt ccactgcgcc c 31

<210> 7
<211> 8
<212> PRT
<213> Artificial Sequence

<220>
<223> Octaarginine (R8) cell-penetrating peptide sequence

<400> 7

Arg Arg Arg Arg Arg Arg Arg Arg
1               5

<210> 8
<211> 27
<212> DNA
<213> Artificial Sequence

<220>
<223> Artificially synthesized primer sequence (EcoR1-Cya1200R)

<400> 8
tcgaattcct ggcgttccac tgcgccc 27

<210> 9
<211> 28
<212> DNA
<213> Artificial Sequence

<220>
<223> Artificially synthesized primer sequence (EcoRI-sGFP-F)

<400>  9
taggaattca tggtgagcaa gggcgagg                                          28

<210>  10
<211>  32
<212>  DNA
<213>  Artificial Sequence

<220>
<223>  Artificially synthesized primer sequence (XhoI-sGFP-R)

<400>  10
agtctcgagt tacttgtaca gctcgtccat gc                                     32

## Description

### [Technical Field]

The present invention relates to a method for introducing a substance such as a protein or a nucleic acid into a plant cell using plasma.

### [Background Art]

Introduction of a substance such as a protein or a nucleic acid into a cell is of great significance not only in basic research but also in various industrial applications.

For the introduction of a substance into a mammalian cell, a method utilizing cell permeability factors and the like involved in cell endocytosis and intracellular invasion of microorganisms has already been established and widely used. In addition, various transfection reagents are commercially available, and appropriate selection and use of them make it possible to introduce the above substances into mammalian cells.

On the other hand, such transfection method is not very effective on plant cells, and it is difficult to introduce the above substances to the plant cells. This is presumably because the plant cell is wrapped in a strong cell wall containing cellulose, and this cell wall blocks the introduction of the substance.

For such substance introduction into plant cells, there have been tried methods including a method for treating the cell wall with an enzyme such as cellulase (protoplast method), a method for introducing a substance into a cell using a fine syringe (microinjection method), a method for covering metal fine particles with a substance and shooting them into cells (gene gun method), and a method for electrically punching holes in a cell membrane and allowing a substance to flow into the cell (electroporation method).

However, the introduction by these methods requires appropriate conditions to be considered for each plant species, and there are many plant species for which introduction conditions have not been established yet. In addition, introduction by these methods involves a cumbersome and laborious operation and requires time. Moreover, the above methods are poor in terms of introduction efficiency, and further cause damage (damae) to the plant cell of introduction target. As for the protoplast method, isolation of protoplasts is difficult, and there are many cases where it is difficult to cultivate them and redifferentiate them to whole plant. Therefore, in view of such problems, there is a demand for a method which makes it possible to easily and highly efficiently introduce a substance into a plant cell without causing a damae regardless of the types of the plant and tissue from which the plant cell is derived. However, no such method has been established yet.

Meanwhile, plasma treatment, particularly atmospheric pressure nonthermal plasma treatment, is drawing attention in various fields, for example the manufacturing industry, the pharmaceutical industry, and environmental control. Indeed, the present inventors have revealed that such a plasma treatment is effective for the hydrophilic treatment of polyimide film surfaces. The present inventors have also reported that it is possible to decompose anesthetic gases and toxic chemicals with atmospheric pressure plasma. Moreover, regarding the effects on living organisms, the present inventors have also clarified that bacteria and biomolecules are inactivated by atmospheric pressure nonthermal plasma.

In addition, regarding the introduction of a substance into a cell using plasma treatment, it has been reported that a substance was introduced into a mammalian cell by irradiating the cell with plasma in the presence of the substance (Patent Literatures 1 and 2). Here, Patent Literature 2 states that such plasma treatment usually causes a damae to cells, and the same literature also shows that, for example the number of survived cells after irradiation was reduced to half or less (see the description of the paragraph [0076] of Patent Literature 2). In addition, the same literature also shows that, in the introduction of a protein into a mammalian cell by plasma treatment, introduction efficiency thereof is improved in the presence of a CPP (see the description of the paragraph [0082] of Patent Literature 2).

However, regarding plant cells, no method has been established yet which easily and highly efficiently introduces a substance into a plant cell without causing a damae regardless of the types of the plant and tissue from which the plant cell is derived, as described above.

### [Citation List]

### [Patent Literature]

[PTL 1] International Publication No. WO 2002/064767
[PTL 1] International Publication No. WO 2011/148996

### [Summary of Invention]

### [Technical Problem]

The present invention has been made in view of the problems of the related art, and aims to provide a method for easily and highly efficiently introducing a substance into a plant cell without causing a damae regardless of the types of the plant and tissue from which the plant cell is derived.

### [Solution to Problem]

The present inventors have made earnest studies and revealed as a result that when a substance such as a protein or a nucleic acid is brought into contact with a plasma-treated plant tissue, the substance is introduced into the cells of the plant. Cells are treated with plasma in the presence of a substance in Patent Literatures 1 and 2. Surprisingly, however, even when a substance was brought into contact with a plant cell after a while subsequent to plasma treatment, the substance was successfully introduced into the cell. In addition, although it is generally difficult to introduce a substance into a plant cell compared with a mammalian cell because of a cell wall, the above method made it possible to introduce a substance into a plant cell without using a cell penetrating peptide (CPP) or the like. Additionally, the present inventors have also found that there are no restrictions on the types of plants and tissues capable of substance introduction, and moreover that plasma treatment does not cause a damae to plant cells. These findings have led to the completion of the present invention.

Specifically, the present invention relates to a method for introducing a substance such as a protein or a nucleic acid into a plant cell using plasma, and more specifically provides the following.
<1> A method for introducing a substance into a plant cell, comprising: treating the cell with plasma; and then bringing the substance into contact with the cell.
<2> The method according to <1>, wherein the substance is a protein or a nucleic acid.
<3> The method according to <1> or <2>, wherein the plasma is normal-temperature atmospheric pressure plasma.
<4> The method according to any one of <1> to <3>, wherein the plasma is at least one plasma selected from the group consisting of carbon dioxide plasma, nitrogen plasma, oxygen plasma, hydrogen and argon mixture plasma, and air plasma.
<5> The method according to any one of <1> to <3>, wherein the plasma is at least one plasma selected from the group consisting of carbon dioxide plasma and nitrogen plasma.

Note that in the present invention, "carbon dioxide plasma," "nitrogen plasma," and the like are names based on the type of gas used for generating each plasma (carbon dioxide, nitrogen, and the like).

### [Advantageous Effects of Invention]

The present invention makes it possible to easily and highly efficiently introduce a substance into a plant cell without causing a damae regardless of the types of the plant and tissue from which the plant cell is derived.

### [Brief Description of Drawings]

Fig. 1 is a schematic diagram showing an embodiment of plasma treatment according to the present invention, used in Examples. Specifically, Fig. 1 is a diagram which shows that a plasma generation gas is allowed to flow into a plasma generator 1 from a gas supplier 2 and simultaneously the gas is cooled by a gas cooler 4, and thereafter a power supplier 3 applies a voltage on an internal electrode in the plasma generator to irradiate a sample 6 (a plant cell, for example a tobacco leaf explant) with plasma 5.

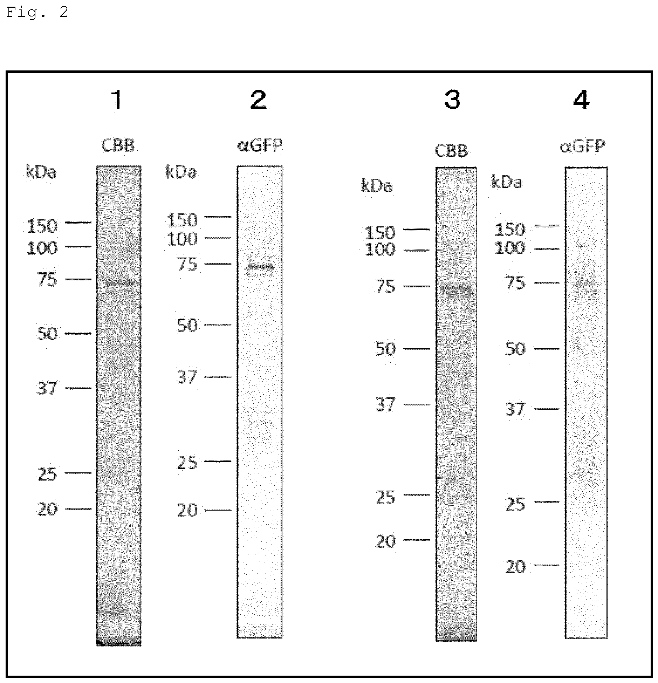

Fig. 2 provides photos showing the results in which a His-tag fusion protein expressed in E. coli and purified using a nickel affinity chromatography support was developed by SDS-PAGE and analyzed by CBB staining and immunoblot. In the figure, "1" indicates the result of analyzing a His-tag fused sGFP-CyaA protein by CBB staining, "2" indicates the result of analyzing a His-tag fused sGFP-CyaA protein by immunoblot using an anti-GFP antibody, "3" indicates the result of analyzing a His-tag fused sGFP-CyaA-R8 protein by CBB staining, and "4" indicates the result of analyzing a His-tag fused sGFP-CyaA-R8 protein by immunoblot using an anti-GFP antibody. Note that the amount of protein used for these analyses was 1 µg in CBB staining and 50 ng in immunoblot.
Fig. 3 provides photos showing the results of confocal microscope observation of a tobacco leaf explant subjected to irradiation with carbon dioxide plasma, oxygen plasma, hydrogen and argon mixed gas plasma, or nitrogen plasma followed by contact with a His-tag fused sGFP-CyaA-R8 protein.

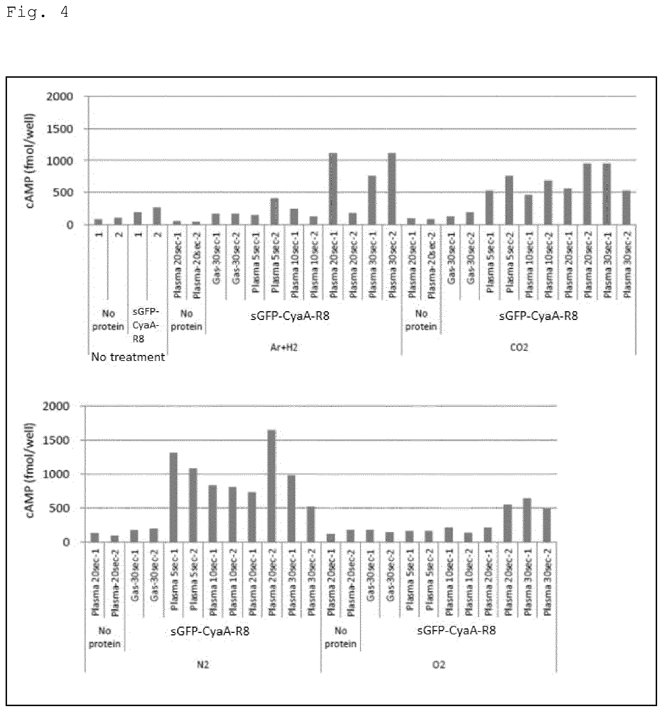

Fig. 4 provides graphs showing the results of measuring the amount of cAMP produced in a tobacco leaf explant subjected to irradiation with hydrogen and argon mixture gas plasma, carbon dioxide plasma, nitrogen plasma, or oxygen plasma followed by contact with a His-tag fused sGFP-CyaA-R8 protein. Note that the figure provides results of two leaf explants tested independently under each set of conditions. In addition, in the figure, "No protein" simply indicates the result of contact with a PBS solution (containing no protein), and "No treatment" indicates the result of explant without plasma treatment.
Fig. 5 provides photos showing the appearance of a tobacco leaf explant 6 days after irradiation with carbon dioxide plasma or nitrogen plasma. In the figure, "2 sec" and "5 sec" indicate plasma irradiation period. The scale bar in the figure indicates 1 cm.
Fig. 6 provides photos showing the results of confocal microscope observation of a tobacco leaf explant subjected to irradiation with carbon dioxide plasma or nitrogen plasma followed by contact with a His-tag fused sGFP-CyaA protein.
Fig. 7 provides graphs showing the results of measuring the amount of cAMP produced in a tobacco leaf explant subjected to irradiation with carbon dioxide plasma or nitrogen plasma followed by contact with a His-tag fused sGFP-CyaA protein.
Fig. 8 provides photos showing the results of confocal microscope observation of a tobacco leaf explant subjected to irradiation with air plasma followed by contact with a His-tag fused sGFP-CyaA protein.
Fig. 9 provides photos showing the results of confocal microscope observation of a leaf of Arabidopsis thaliana or a root of rice subjected to irradiation with carbon dioxide plasma or nitrogen plasma followed by contact with a His-tag fused sGFP-CyaA protein.
Fig. 10 provides photos showing the results of confocal microscope observation of a tobacco leaf explant subjected to irradiation with carbon dioxide plasma followed by contact with a solution of plasmid DNA encoding an sGFP protein. The scale bar in the figure indicates 50 µm.

### [Description of Embodiments]

### (Method for Introducing Substance into Plant Cell)

A method for introducing a substance into a plant cell of the present invention is a method comprising: treating the cell with plasma; and then bringing the substance into contact with the cell.

In the present invention, the "plant" is not particularly limited, and examples thereof include angiosperms encompassing dicotyledonous plants (tobacco, Arabidopsis thaliana, and the like) and monocotyledonous plants (rice and the like), gymnosperms, bryophytes, fern plants, herbaceous plants, and woody plants.

As the "plant cell," plant cells present in any tissue or plant cells derived from any tissue can be used in the present invention and are not particularly limited. Examples of such tissue include leaves, roots, root ends, anthers, flowers, seeds, pods, stems, shoot apexes, embryos, and pollens. Also, the method of the present invention can include artificially treated plant cells (for example, calluses and suspension cultured cells) can also be targeted.

Examples of the "substance" to be introduced to the plant cell described above include, but not particularly limited to, biopolymers such as nucleotides (DNA and RNA), peptides, sugars, and lipids. Here, examples of the nucleotides include oligonucleotides, polynucleotides, and nucleic acids, examples of the peptides include oligopeptides, polypeptides, and proteins, and examples of the sugars include oligosaccharides and sugar chains. In addition, examples of the "substance" according to the present invention include not only naturally occurring biopolymers but also derivatives thereof (for example, crosslinked nucleotides and unnatural amino acids) as well as complexes thereof (for example, glycoproteins, glycolipids, and RNA-protein complexes).

In the present invention, "plasma" includes a charged particle group in which molecules constituting a gas are divided into positive (positive ions) and negative (electrons) by ionization, and means a group of particles (ionized gas) which is electrically almost neutral as a whole. The "plasma" for treating plant cells are not particularly limited, and may be generated under atmospheric pressure (atmospheric pressure plasma) and may be generated under a pressure lower than the atmospheric pressure (low pressure plasma) . However, the atmospheric pressure plasma is preferable from the viewpoints that a vacuum system is not required for its generation and that it is close to the living environment of plants. Note that in the present invention, the atmospheric pressure does not necessarily have to be strictly 1013 hPa, but may be in the vicinity of the pressure (700 to 1300 hPa).

No particular limitation is imposed on the method for generating atmospheric pressure plasma, and those skilled in the art can appropriately perform the generation using a known method. Examples of the known method include dielectric barrier discharge, inductively coupled plasma discharge (ICP), capacitively coupled plasma discharge (CCP), hollow cathode discharge, corona discharge, streamer discharge, glow discharge, and arc discharge. Preferable among these are glow discharge and hollow cathode discharge from the viewpoints that relatively high plasma, electron, and radical densities can be obtained and the temperature of the plasma gas can be kept low.

In addition, although it cannot be said that current for causing the discharge is unique because it depends e.g. on the type of the discharge, the size and shape of the apparatus for generating the discharge (and hence plasma), and the size and shape of the electrodes for applying a voltage in order to generate the discharge, the current for causing the discharge may be a direct current or an alternating current.

The temperature of the "plasma" for treating the above plant cells is not particularly limited, and is usually -90 to 200°C, preferably 0 to 50°C, and more preferably 20 to 30°C (room temperature). Such temperature control can be achieved by the method described in, for example, Oshita T, Kawano H, Takamatsu T, Miyahara H, Okino A (2015) "Temperature Controllable Atmospheric Plasma Source" IEEE Trans Sci43: 1987-1992 and Japanese Unexamined Patent Application Publication No. 2010-061938. More specifically, the method makes it possible to control temperature of plasma toward the desired value by a step of 1°C as follows: the gas used for generating plasma to be described later is cooled to a low temperature (for example, -195°C) by a gas cooler using liquid nitrogen or the like. Thereafter, the gas is heated to a desired temperature with a heater to form plasma, and further the gas temperature of the generated plasma is fed back to the heater.

No particular limitation is imposed on the type of gas to which a voltage is applied in order to generate plasma, but it is preferably at least one gas selected from carbon dioxide, nitrogen, oxygen, hydrogen, and argon, and more preferably carbon dioxide and nitrogen from the viewpoint of introduction efficiency of the substance. In addition, as shown in Examples to be described later, a mixture gas composed of hydrogen and argon (preferably having a volume percentage of 0.01 to 50% hydrogen and 99.99 to 50% argon) and a mixture gas composed of nitrogen and oxygen (what is called air, preferably having a volume percentage of 90 to 70% nitrogen and 30 to 10% oxygen) are also preferably used.

Additionally, the rate of flow of the gas supplied to the plasma generator can be appropriately adjusted by those skilled in the art to, for example, 3 to 5 L/min in consideration of the size, shape, and the like of the apparatus and moreover the stabilization of plasma generation while avoiding the situation where the sample (plant cell) is blown away by the current of the plasma.

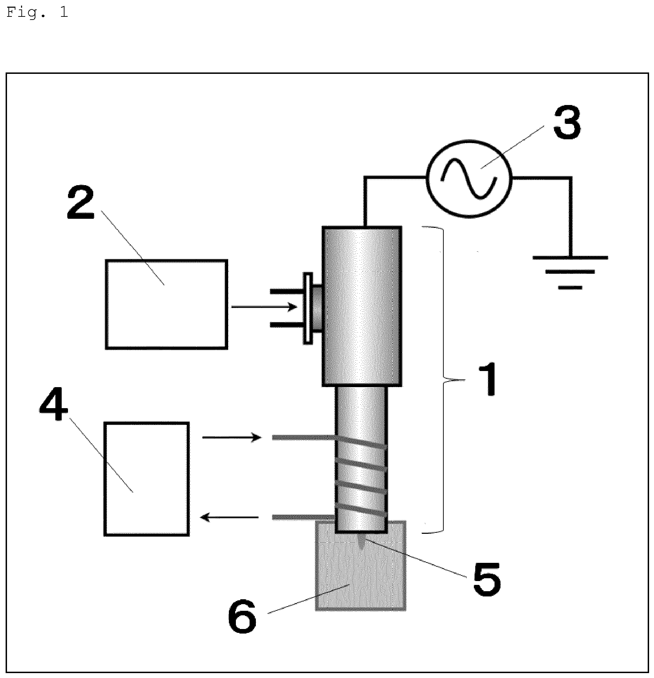

Such apparatus for generating plasma is not particularly limited, but a configuration as shown in Fig. 1 is presented, for example. More specifically, the apparatus for introducing a substance into a plant cell of the present invention preferably includes at least a plasma generator 1 capable of generating plasma as well as an apparatus (gas supplier 2) for supplying the generator with the gas used to generate plasma and an apparatus (power supplier 3) for supplying power to ionize the gas. It is more preferable to further include a gas cooler 4 for controlling the temperature of plasma and/or a table (mount table) for mounting a sample 6 (plant cell) . Moreover, although not shown in Fig. 1, it is further preferable to provide a gas cooling and gas heating system (gas temperature adjustment system) instead of the gas cooler 4 between the gas supplier 2 and the plasma generator 1. Furthermore, although not shown in Fig. 1, a heat insulating material may be provided instead of the gas cooler 4 in order to avoid the inflow of heat from the outside. Note that the plasma generator is not particularly limited, and a known apparatus may be appropriately used. For example, preferably used in the present invention are the apparatuses disclosed in Japanese Unexamined Patent Application Publication No. 2015-072913, Japanese Unexamined Patent Application Publication No. 2014-212839, Japanese Unexamined Patent Application Publication No. 2013-225421, Japanese Unexamined Patent Application Publication No. 2013-094468, Japanese Unexamined Patent Application Publication No. 2012-256501, Japanese Unexamined Patent Application Publication No. 2008-041429, Japanese Unexamined Patent Application Publication No. 2009-082796, and JP 2010-061938 A.

The treatment of a plant cell with plasma generated as described above is usually accomplished by placing the cell below the plasma irradiation port followed by irradiation with plasma. In that case, the irradiation time is not particularly limited and can be appropriately adjusted depending on the types of plasma and plant cell used. However, the irradiation time is preferably 0.01 to 3 minutes, more preferably 1 to 30 seconds, further preferably 1 to 10 seconds, and particularly preferably 2 to 5 seconds from the viewpoint of further enhancing the introduction efficiency of the substance while suppressing the damae in the plant cell.

Furthermore, although the distance from the plasma irradiation port to the plant cell is not particularly limited either, it is desirable to keep the distance from the plasma irradiation port as short as possible because the plasma starts deactivation immediately after leaving the plasma generation section. On the other hand, plasma is required to be exhausted as a gas flow, and it is also desirable to uniformly irradiate the plant cell while suppressing the plant cell from being blown away. In addition, those skilled in the art can appropriately adjust the distance so as to comply with the above-mentioned viewpoints, taking into consideration the plasma apparatus to be used and the gas flow thereof as well as the type and size of the plant cell, and the like. For example, the distance from the plasma irradiation port to the plant cell is about 5 to 7 mm.

The contact initiation time between the plant cell treated with plasma in this way and the substance to be introduced into the cell is not particularly limited, but is preferably between 0.01 to 30 minutes and more preferably 0.01 to 5 minutes after the treatment with plasma from the viewpoint of further enhancing the substance introduction efficiency by plasma treatment. Furthermore, the contact time between the plant cell and the substance is not particularly limited either, but is preferably 1 minute to 30 hours from the viewpoint of the introduction efficiency of the substance and the subsequent normal growth of the plant cell.

No particular limitation is imposed on the method for bringing the substance into contact with the plasma-treated plant cell, and the substance itself may be directly added to the plasma contact portion of the plant cell. However, depending on the properties of the substance, the substance may be supported, added, mixed, or contained in a support for promoting the introduction whereby to add the substance. Examples of such support include phospholipid compositions such as liposomes, metals (gold, tungsten, and the like), particles made of inorganic matters (silicon compounds and the like), whiskers, alginate beads, viral agents (for example, coat proteins), and cell penetrating peptides (CPP).

Moreover, the plant cell and the substance can be brought into contact together by addition to a solution containing the substance (or e.g. a mixture of the substance and a support) or immersion of the plant cell in the solution. Such a solution is not particularly limited as long as it can keep the plant cell alive, and examples thereof include buffer solutions (for example, phosphate buffered saline (PBS), phosphate buffer solutions (NaPO₄ and KPO₄), HEPES buffer solutions, tris buffer solutions, MES buffer solutions, and citrate buffer solutions) and culture media (for example, Murashige & Skoog (MS) medium). Additionally, the concentration of the substance in the solution can be appropriately adjusted depending on the type of each substance, the types of the plant to be introduced and the tissue thereof, and the like. The concentration is usually 1 to 100 µg/ml when the substance is a protein, and the concentration is usually 1 to 100 µg/ml when the substance is a DNA.

### [Examples]

Hereinafter, the present invention will be described in more detail based on Examples, but the present invention is not limited to Examples below. In addition, the experiments to be described later were conducted using the materials and methods described below.

### (Plant)

Plant tissues to be subjected to e.g. plasma treatment of present invention were prepared as follows.

For tobacco (Nicotiana tabacum cv.Samsun NN), the seeds were sowed on soil and cultivated at 25°C under a cycle of light 16 hours/dark 8 hours. Leaves (mature leaves) 4 to 8 weeks after sowing were cut on a paper towel into square pieces of about 1.5 to 2 cm, and were subjected to the plasma treatment to be described later. In addition, for the maintenance culture of the leaf explants, a plate medium with a 1/2 salt concentration of Murashige & Skoog (MS) was used.

Rice (Nipponbare) underwent hydroponic cultivation using tap water at 27°C under a cycle of light 16 hours/dark 8 hours. Roots 2 to 3 weeks after sowing were cut on a slide to have a length of about 0.5 to 3 cm, and were subjected to the plasma treatment to be described later.

For Arabidopsis thaliana (Col-0), the seeds were sowed on soil and cultivated at 22°C under a cycle of light 12 hours/dark 12 hours. Leaves (mature leaves) 4 to 8 weeks after sowing were cut off and subjected to the plasma treatment to be described later as they were.

### (Preparation of sGFP-CyaA Fusion Protein)

The proteins introduced into the plant tissue by the plasma treatment of the present invention were prepared as follows.

Specifically, first, a protein (His-tag fused sGFP-CyaA protein) prepared by fusing adenylate cyclase (CyaA), a superfolder green fluorescent protein (sGFP), and a His tag was prepared. More specifically, a DNA fragment encoding sGFP (SEQ ID NO: 1) was amplified by PCR using the BamHI-sGFP-F primer (5'-TAGGATTCACCATGGTGAGCAAGGGCGAGG-3', SEQ ID NO: 2) and the EcoRI-sGFP-R primer (5'-TAGAATTCCTTGTACAGCTCGTCCATGCCG-3', SEQ ID NO: 3) with pGWB5 (see Nakagawa T et al., (2007), Journal of Bioscience and Bioengineering 104, 34-41) as a template. In addition, in order to prepare the pENTR3C-sGFP vector, the fragment obtained by the above amplification was treated with BamHI and EcoRI and then inserted into the pENTR3C vector (manufactured by Invitrogen). Next, in the open reading frame of CyaA (ORF, SEQ ID NO: 4), the portion encoding the N-terminal 400 amino acids was amplified by PCR using the EcoRI-Cya-F primer (5'-TAGAATTCATGCAGCAATCGCATCAGGC-3', SEQ ID NO: 5) and the XhoI-stop-Cya1200R primer (5'-TCACTCGAGCTACTGGCGTTCCACTGCGCCC-3', SEQ ID NO: 6) with pHMCyA (see Furutani A et al., Mol Plant Microbe Interact. (2009) Jan; 22(1): 96-106) as a template. The fragment amplified in this way was treated with EcoRI and XhoI and inserted into pENTR3C-sGFP to prepare pENTR3C-sGFP-CyaA. Subsequently, the plasmid was treated with BamHI and XhoI. After that, in order to fuse 6 × histidine (His tag) to the N-terminal of sGFP-CyaA, the treated fragment (sGFP-CyaA fragment) was inserted into the pET28a vector (manufactured by Novagen) to prepare the pET28a-sGFP-CyaA plasmid.

In addition, in order to further prepare a His-tag fused sGFP-CyaA-R8 protein obtained by fusing a cell penetrating peptide, namely arginine 8 amino acid (R8, SEQ ID NO: 7), a DNA fragment encoding R8 was prepared by annealing the single-stranded DNAs EcoRI-R8-stop-XhoI-F and XhoI-stop-R8-EcoRI-R, and inserted into pENTR3C-sGFP treated with EcoRI and XhoI. The ORF encoding the N-terminal 400 amino acids of CyaA was amplified by PCR using EcoRI-Cya-F primer and EcoRI-Cya1200R primer (5'-TCGAATTCCTGGCGTTCCACTGCGCCC-3', SEQ ID NO: 8) with pHMCyA as a template. The fragment amplified in this way was treated with EcoRI and inserted into pENTR3C-sGFP-R8 treated with EcoRI. Next, the plasmid obtained in this way was further treated with BamHI and XhoI. Thereby, the sGFP-CyaA-R8 fragment was excised and replaced with the pET28a vector to prepare the pET28s-sGFP-CyaA-R8 plasmid.

The plasmids pET28a-sGFP-CyaA and pET28a-sGFP-CyaA-R8 prepared as described above were introduced into E. coli BL21 (DE3). Then, these E coli were cultured to express the fusion proteins His-tag fused sGFP-CyaA and His-tag fused sGFP-CyaA-R8 encoded by these plasmids. Thereafter, a chromatographic resin for purification of His-tag proteins (manufactured by GE Healthcare, trade name: Ni Sepharose High Performance) was used for purification by the method described in the manual.

Note that it has been confirmed by CBB staining and immunoblot using anti-GFP antibody (produced by Abcam) that these purified proteins are the desired fusion proteins (see Fig. 2).

### (Plasma Treatment)

The plasma treatment was conducted in accordance with the methods described in Takamatsu T, Hirai H, Sasaki R, Miyahara H, Okino A (2013) "Surface Hydrophilization of Polyimide Films Using Atmospheric Damage-Free Multigas Plasma Jet Source" IEEE Trans. Plasma Sci 41: 119-125 and Oshita T, Kawano H, Takamatsu T, Miyahara H, Okino A (2015) "Temperature Controllable Atmospheric Plasma Source" IEEE Trans Sci43: 1987-1992.

More specifically, as shown in Fig. 1, the apparatus main body of the plasma generator (Damage-Free Multigas Plasma Jet manufactured by Plasma Concept Tokyo Inc. (damage-free plasma (Japanese registered trademark No. 5409073)) and Multigas Plasma ((Japanese registered trademark No. 5432585), product number: PCT-DFMJ02)) was grounded, and a certain high voltage was supplied via an internal high-voltage electrode of the plasma generation section from the apparatus main body. The certain high voltage is a modulated alternating voltage of 10 to 30 kHz with a maximum of 9 kV. Such electric power is supplied to the plasma generation section to generate glow discharge. Further, argon, hydrogen, carbon dioxide, nitrogen, oxygen, air, and a mixture gas thereof as gas species passed through a 1-mm hole at a rate of flow of 5 L/min to generate stable atmospheric pressure plasma.

Note that the temperature of the plasma generated in this way (the temperature at 5 mm away from the plasma irradiation port) was 50°C or less as a result of thermocouple measurement. In order to generate plasma having a lower temperature (about 20 to 30°C), the gas was cooled by a gas cooling apparatus using liquid nitrogen.

Then, the irradiation port was set at a position 5 mm vertically above the plant tissue prepared as described above, followed by plasma treatment. Thereafter, a PBS solution containing or not containing the purified fusion protein was brought into contact with the plant tissue.

### (cAMP Enzyme Immunoassay)

The CyaA protein is an enzyme which catalyzes the production of the cyclic AMP (cAMP) in a manner dependent on ATP and calmodulin protein present in the cytoplasm. For this reason, when a fusion protein containing CyaA is introduced into a cell, the amount of the protein introduced can be evaluated by measuring the amount of cAMP in the cell.

Therefore, in order to quantitatively analyze the fusion protein introduced into the plant tissue by the above plasma treatment, the amount of cAMP was measured using cAMP Biotrack Enzyme Immunoassay (EIA) System (produced by Amersham) in accordance with the attached manual.

More specifically, leaves of tobacco were treated in accordance with the method of the present invention, and then leaf disks having a diameter of 13 mm were prepared from the leaves. The leaf disks were ground with liquid nitrogen in a pestle and mortar, and further, the resulting powder was treated with 320 µL of 6% (w/v) trichloroacetic acid. Next, 200 µL of the homogenate was centrifuged at 4°C and 2000 g for 15 minutes. The resultant supernatant was washed 4 times with 5 volumes of diethyl ether saturated with water. Next, the remaining water extract was dried with a vacuum dryer at 55°C. Then, the dried extract was dissolved in 200 µL of assay buffer attached to the kit, and 40 µL of each dissolved extract was subjected to cAMP enzyme immunoassay.

### (Confocal Microscope)

In order to analyze the fusion protein introduced into the plant tissue, fluorescence emitted by GFP contained in the protein was detected. Specifically, GFP images, intrinsic fluorescence, and bright-field images were acquired using a confocal laser scanning microscope FV-300 and Fluoroview software (both manufactured by Olympus Corporation).

### (Example 1)

### Introduction of Protein to Tobacco Leaf by Plasma Treatment

Tobacco leaves were treated with plasma (irradiation time: 2 to 30 seconds) using a low temperature (20 to 30°C) multigas plasma jet. One to five seconds after the irradiation, the tobacco leaves were floated on a PBS solution containing a His-tag fused sGFP-CyaA-R8 protein, and incubated (incubation time: 12 to 24 hours, concentration of protein solution: 50 µg/ml, and volume of solution: 400 µl). Then, 12 to 24 minutes after the incubation, a fluorescence signal derived from GFP protein was detected with a confocal microscope. Note that the gas source of the multigas plasma jet used was CO₂, O₂, a mixture gas of H₂ and Ar (volume percentage: 5% H₂ and 95% Ar), and N₂.

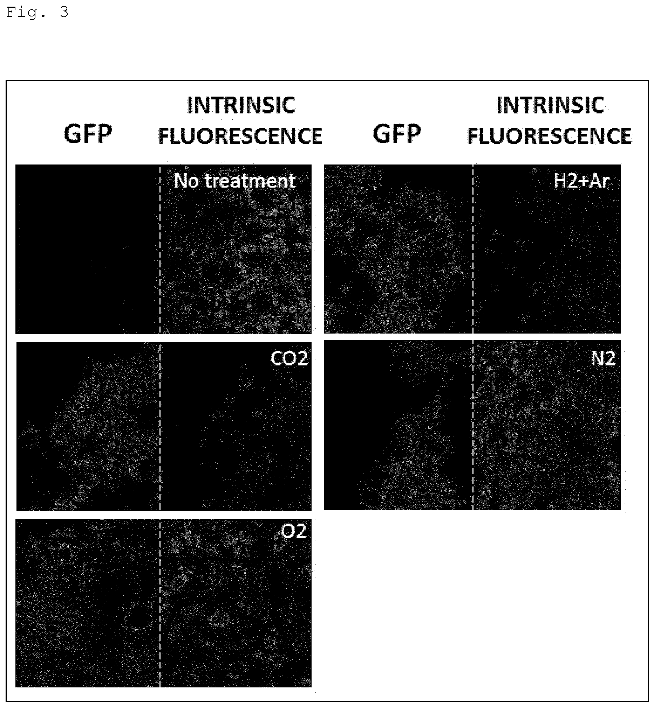

As a result, as shown in Fig. 3, it was revealed that use of any gas source makes it possible to introduce a protein into tobacco leaves by plasma treatment. Particularly surprisingly, it was revealed that the substance is introduced into the plant cell even though the substance is brought into contact with the cell some time after the plasma treatment instead of treating the cell with plasma in the presence of the substance as disclosed in Patent Literatures 1 and 2.

Next, in order to further select a gas source with high introduction efficiency, the tobacco leaves were treated with the plasma generated using CO₂, O₂, a mixture gas of H₂ and Ar, and N₂ as a gas source as described above, and the amount of cAMP in the leaves was quantitatively analyzed.

As a result, similarly to Fig. 3, it was confirmed as shown in Fig. 4 that the protein had been introduced into tobacco leaves by performing plasma treatment using any gas source. In particular, treatment with plasma generated from CO₂ or N₂ significantly increased the amount of cAMP regardless of the treatment time compared with the amount of cAMP in the case of treatment with those gases (control). Regarding O₂ and a mixture gas of H₂ and Ar, it was observed that treatment with the generated plasma for 20 seconds or 30 seconds increased the amount of cAMP, but for the treatment time of 5 seconds or 10 seconds, there was a tendency that a difference from the control was not easily recognized.

### (Example 2)

### Verification of Effects of Plasma Treatment on Plant Cell

It has been revealed that plasma generated from CO₂ or N₂ is capable of inactivating microorganisms (see Takamatsu T, Uehara K, Sasaki Y, Hidekazu M, Matsumura Y, Iwasawa A, Ito N, Kohno M, Azuma T, Okino A (2015) "Microbial Inactivation in the Liquid Phase Induced by Multigas Plasma Jet" PLoS One 10: e0135546).

In addition, it has been reported that a substance was introduced into a mammalian cell by irradiating the cell with plasma in the presence of the substance. However, plasma treatment usually causes a damae to cells: for example, it has been shown that the survival rate of cells after irradiation is less than half (see the description of the paragraph [0076] of Patent Literature 2). Therefore, investigation was conducted on whether or not these plasmas would cause damage to the plant tissue. Specifically, tobacco leaves were treated with CO₂ plasma or N₂ plasma for 2 seconds or 5 seconds, and thereafter the morphology in the leaves was observed for 6 days.

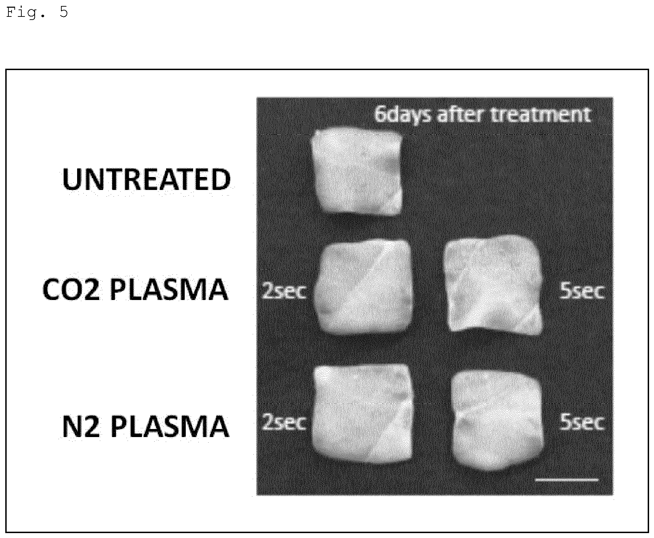

As a result, as shown in Fig. 5, no significant damage was observed in the tobacco leaves even after 6 days following the plasma treatment. Therefore, it was revealed that such plasma treatment does not cause a damae to plant tissues.

### (Example 3)

### Introduction of Protein into Tobacco Leaf by Plasma Treatment without Using CPP

Regarding the introduction of a substance into a plant cell, protein introduction was particularly difficult prior to the filing of the present application. It has been reported that a protein was successfully introduced into, for example, cells of the shoot apical meristems of a plant by exposing the cells to a suspension of a cell penetrating peptide (CPP) and e.g. florigen protein (see International Publication No. WO 2013/118863). Moreover, it has been reported that infiltration using a syringe made it possible to introduce a complex of a protein, a CPP containing a polycation sequence, and the like into a plant cell (see Ng KK et al., (2016), PLoS One 11: e0154081 and International Publication No. WO 2013/129698). It has also been shown that a CPP promotes the efficiency of introducing a substance into a mammalian cell by plasma treatment (see the description of the paragraph [0082] of Patent Literature 2).

In light of the above, investigation was conducted on whether or not a CPP is necessary when a protein is introduced into a plant cell by plasma treatment as follows. Specifically, tobacco leaf explants were treated with CO₂ plasma or N₂ plasma and then floated on a PBS solution containing a His-tag fused sGFP-CyaA protein instead of a PBS solution containing the His-tag fused sGFP-CyaA-R8 protein described above for incubation. In this way, the presence or absence of the introduction of the fusion protein was detected.

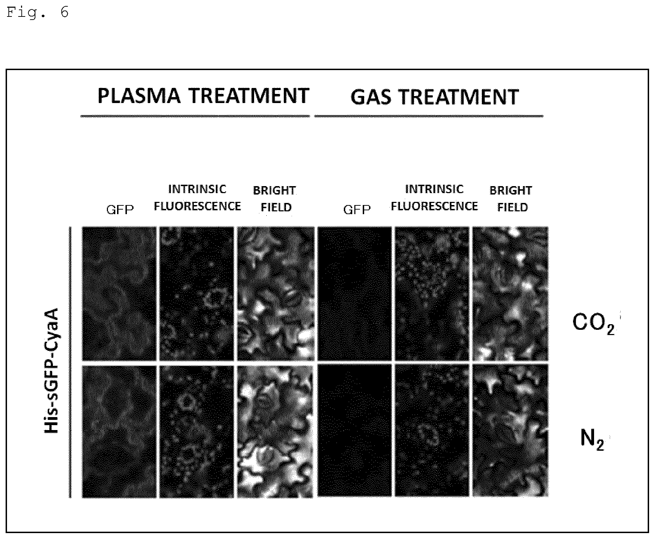

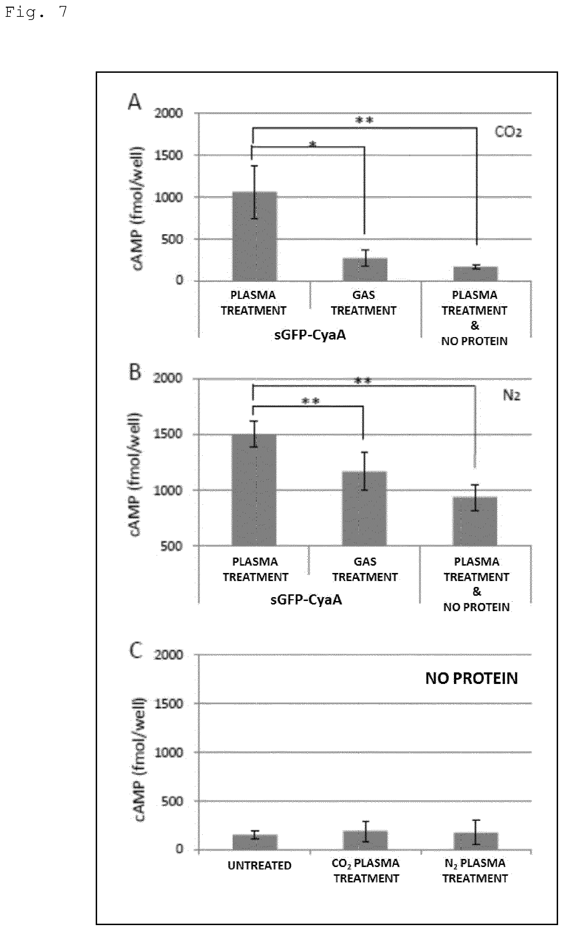

As is apparent from the results shown in Fig. 6, fluorescence derived from GFP was detected in any of the cells treated with CO₂ plasma and N₂ plasma. Moreover, as shown in Fig. 7, CO₂ plasma treatment significantly increased the amount of cAMP by about 4.0 times as compared with the treatment with the gas. In addition, N₂ plasma treatment increased the amount of cAMP by about 1.3 times as compared with the treatment with the gas.

Note that the amount of cAMP was measured in the leaf explants to which no protein was added after plasma treatment in order to confirm that the amount of cAMP actually increased due to the introduction of His-sGFP-CyaA by plasma treatment. As a result, as expected, there was no significant difference between plasma-treated and untreated results (see Fig. 7C).

From the above results, it was revealed that a CPP is not necessary for introduction of a protein into a plant cell by plasma treatment. Particularly surprisingly, it was revealed that such method makes it possible to introduce a substance into a plant cell without using a CPP, although substance introduction is difficult as compared with a mammalian cell because plant cells have cell walls.

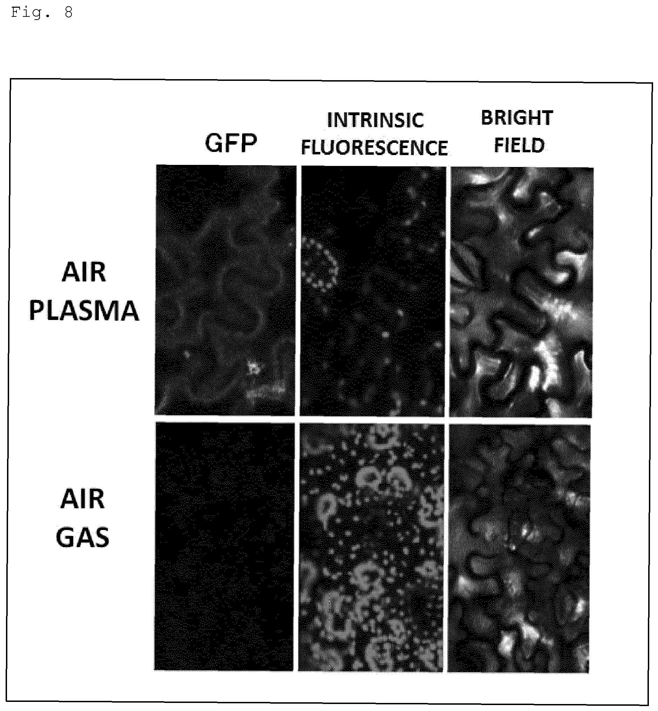

In addition, the gas species was changed from CO₂ and N₂ to air (volume percentage: 80% N₂ and 20% O₂), and the presence or absence of introduction of a His-tag fused sGFP-CyaA protein into the plasma-treated tobacco leaves was similarly analyzed. As a result, as shown in Fig. 8, it was revealed that it is possible to introduce a protein into a plant cell without requiring a CPP by air plasma treatment (2 seconds).

### (Example 4)

### Introduction of Protein into Rice Root and Arabidopsis Thaliana Leaf by Plasma Treatment

Protein introduction was attempted by plasma treatment (treatment time: 2 to 5 seconds) of rice roots and Arabidopsis thaliana leaves for the purpose of confirming that a protein can be introduced by plasma treatment into other plants and other tissues as in the case of the tobacco leaves described above. Note that the protein that was attempted to be introduced was a His-tag fused sGFP-CyaA protein.

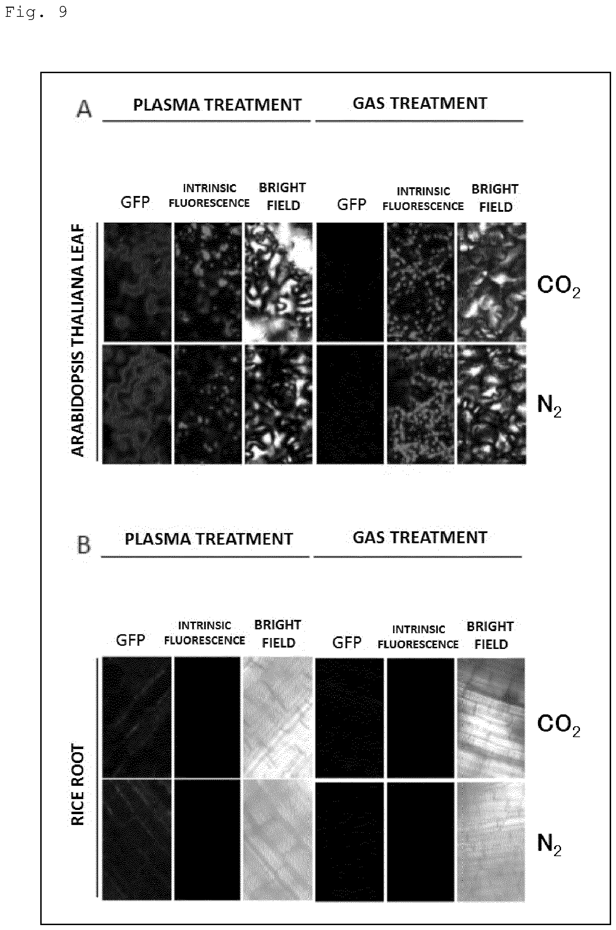

As a result, as is clear from the results shown in Fig. 9, GFP-derived fluorescence was detected in any of the plants and tissues. Therefore, it was confirmed that the method of the present invention makes it possible to introduce a protein regardless of the types of plants and their tissues.

### (Example 5)

Introduction of DNA into Plant cell by Plasma Treatment

As in the case of the above-mentioned protein, it is confirmed that DNA is also introduced into a plant cell by the method of the present invention as described below.

Specifically, plasmid DNA encoding a reporter gene is used as DNA to be introduced into a plant cell. More specifically, examples of the reporter gene used include P_{35S}-sGFP-T_{NOS} encoding green fluorescent protein (sGFP), P_{35S}-GUS-T_{NOS} encoding β-glucuronidase (GUS), and P_{35S}-LUC-T_{NOS} plasmid DNA encoding luciferase (LUC). In addition, here, P_{35S} means a 35S promoter sequence of cauliflower mosaic virus, and T_{NOS} means a terminator sequence of Agrobacterium nopaline synthase gene.

Then, in the same way as in the above-mentioned proteins, a explant was prepared for tobacco leaf, rice root, and Arabidopsis thaliana leaf, and each of the explants was irradiated with N₂ plasma or CO₂ plasma for 2 to 5 seconds . Next, the explants were immersed in a PBS solution containing the plasmid DNA at a concentration of 1 to 100 µg/ml. Thereafter, the explants were placed on an agar medium and maintained at 27°C for 1 to 5 days.

Introduction of plasmid DNA was confirmed as the following indicator. The reporter gene introduced into the cell migrated into the nucleus, and was transcribed and translated to express the protein encoded by the reporter gene in the cell. Expression of sGFP protein was detected by observing a explant maintained at 27°C with a confocal microscope. For expression of GUS protein, a explant maintained at 27°C was treated with X-GLUC, a chromogenic substrate, and observed with a stereoscopic microscope as a blue coloring. For expression of LUC protein, a explant maintained at 27°C was treated with luciferin, a substrate of LUC, and chemiluminescence was detected with a high sensitivity CCD camera (LAS-3000) or the like.

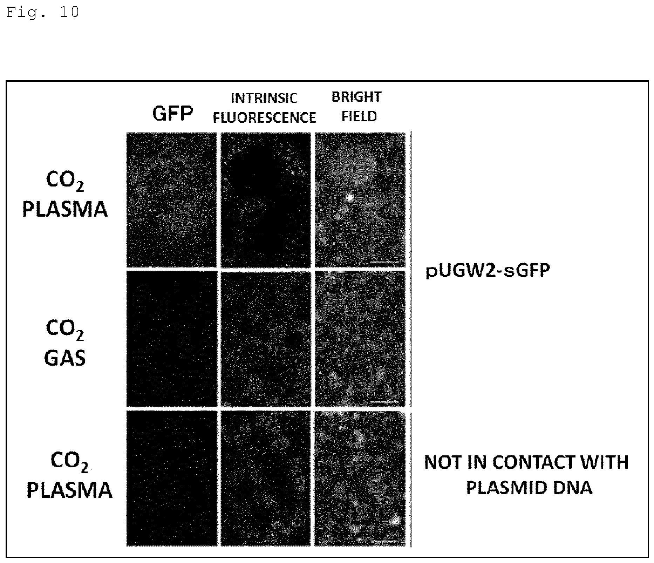

Indeed, in the same way as in the above-mentioned proteins, a explant was prepared for tobacco leaf and the explant was irradiated with CO₂ plasma for 5 seconds. Subsequently, the explant was immersed in a 1/4 × PBS solution containing 20 µg/ml of plasmid DNA (pUGW2-sGFP) to be described later. Three to eight hours later, the explant was placed on callus-forming medium [1 x Murashige & Skoog (MS), 1 × MS vitamin (0.1 µg/ml of thiamine hydrochloride, 0.5 µg/ml of pyridoxine hydrochloride, 0.5 µg/ml of nicotinic acid, 2 µg/ml of glycine, and 100 µg/ml of myoinositol), 0.1 µg/ml of α-naphthaleneacetic acid, 1 µg/ml of 6-benzylaminopurine, 30 g/L of sucrose, 200 µg/ml of cefotax, 8.5 g/L of agar, and pH 5.8] and allowed to stand overnight at room temperature. Thereafter, the explant was transferred under a cycle of light 16 hours/dark 8 hours at 28°C, and further grown for 1 day, and the expression of sGFP protein in the explant was observed with a confocal microscope. In this way, detection was attempted. Also, as a control group, a explant subjected to CO₂ gas treatment instead of the CO₂ plasma treatment and a explant not brought into contact with the following plasmid DNA (pUGW2-sGFP) after CO₂ plasma treatment were also prepared. Attempts were also made in these explants to detect the expression of sGFP protein. The obtained results are shown in Fig. 10.

Note that the introduced plasmid DNA (pUGW2-sGFP) was prepared as follows. A DNA fragment encoding sGFP (SEQ ID NO: 1) was amplified by PCR using the EcoRI-sGFP-F primer (5'-TAGGAATTCATGGTGAGCAAGGGCGAGG-3', SEQ ID NO: 9) and the XhoI-sGFP-R primer (5'-AGTCTCGAGTTACTTGTACAGCTCGTCCATGC-3', SEQ ID NO: 10) with pGWB5 described above as a template. Next, the amplified fragment was treated with EcoRI and XhoI and inserted into the EcoRI and XhoI sites of pENTR3C (produced by Invitorogen-Thermo Fisher Scientific) to prepare a pENTR-sGFP entry clone. Furthermore, in the Gateway LR Clonase reaction, pUGW2-sGFP was prepared by inserting sGFP into the pUGW2 destination vector (see Nakagawa et al (2007) Journal of Bioscience and Bioengineering 104, 34-41) .

As is clear from the results shown in Fig. 10, GFP-derived fluorescence was detected in the tobacco leaf subjected to plasma treatment. Therefore, it was confirmed that the method of the present invention makes it possible to introduce not only a protein but also DNA into a plant cell without special use of CPP or the like.

### [Industrial Applicability]

As described above, the present invention makes it possible to easily and highly efficiently introduce a substance into a plant cell without causing a damae regardless of the types of the plant and tissue from which the plant cell is derived.

Therefore, the method of the present invention is extremely useful in basic research because the functions of the introduced substance (gene, protein, and the like) can be analyzed by a phenotypic change of a plant cell and the like. In addition, a plant cell to which new functions are added by such introduction of a substance is very useful as the fields of production and development of biomass, functional food materials, pharmaceutical materials, and the like. Accordingly, the present invention also makes a great contribution in various industrial applications.

### [Reference Signs List]

1: plasma generator
2: gas supplier
3: power supplier
4: gas cooler
5: plasma
6: sample

### [Sequence Listing Free Text]

SEQ ID NO: 1
   <223> gene sequence of superfolder green fluorescent protein
SEQ ID NO: 2
   <223> sequence of artificially synthesized primer (BamH1-sGFP-F)
SEQ ID NO: 3
   <223> sequence of artificially synthesized primer (EcoR1-sGFP-R)
SEQ ID NO: 4
   <223> adenylate cyclase
SEQ ID NO: 5
   <223> sequence of artificially synthesized primer (EcoR1-Cya-F)
SEQ ID NO: 6
   <223> sequence of artificially synthesized primer (Xho1-Stop-Cya1200R)
SEQ ID NO: 7
   <223> sequence of cell penetrating peptide arginine 8 amino acid
SEQ ID NO: 8
   <223> sequence of artificially synthesized primer (EcoR1-Cya1200R)
SEQ ID NO: 9
   <223> sequence of artificially synthesized primer (EcoRI-sGFP-F)
SEQ ID NO: 10
   <223> sequence of artificially synthesized primer (XhoI-sGFP-R)

## Claims

1. A method for introducing a substance into a plant cell, comprising:
treating the cell with plasma; and
then bringing the substance into contact with the cell.

2. The method according to claim 1, wherein
the substance is a protein or a nucleic acid.

3. The method according to claim 1 or 2, wherein
the plasma is normal-temperature atmospheric pressure plasma.

4. The method according to any one of claims 1 to 3, wherein
the plasma is at least one plasma selected from the group consisting of carbon dioxide plasma, nitrogen plasma, oxygen plasma, hydrogen and argon mixture plasma, and air plasma.

5. The method according to any one of claims 1 to 3, wherein the plasma is at least one plasma selected from the group consisting of carbon dioxide plasma and nitrogen plasma.
